# EUROPÄISCHE PATENTSCHRIFT

(11) **EP 2 717 935 B1**
(45) Veröffentlichungstag und Bekanntmachung des Hinweises auf die Patenterteilung: **07.12.2016**
(21) Anmeldenummer: 12725807.7
(22) Anmeldetag: 06.06.2012
(51) Int. Cl.: A61M 1/00

(54) **WUNDABDECKUNGSARTIKEL MIT VORBEREITUNG ZUM ANSCHLUSS EINER UNTERDRUCKVORRICHTUNG**
WOUND-COVERING ARTICLE WITH PREPARATION FOR ATTACHMENT OF A VACUUM DEVICE
ARTICLE DE RECOUVREMENT DE PLAIE PRÉPARÉ POUR LE RACCORDEMENT À UN DISPOSITIF DE SOUS-PRESSION

(30) Priorität: 07.06.2011 DE 102011050884; 05.01.2012 DE 102012100073; 07.03.2012 DE 102012101906
(43) Veröffentlichungstag der Anmeldung: 16.04.2014
(73) Patentinhaber: BSN medical GmbH, 20253 Hamburg (DE)
(72) Erfinder: RIESINGER, Birgit, 48149 Münster (DE)
(74) Vertreter: Jostarndt Patentanwalts-AG
(86) Internationale Anmeldenummer: PCT/EP2012/060697
(87) Internationale Veröffentlichungsnummer: WO 2012/168298

(56) Entgegenhaltungen:
- EP-A1- 2 098 257
- EP-A1- 2 762 177
- WO-A1-2006/048246
- WO-A1-2006/056294
- WO-A1-2010/094957
- WO-A2-2009/071933
- WO-A2-2009/124125
- DE-U1-202006 007 877
- US-A1- 2008 208 171
- US-A1- 2009 312 723

## Beschreibung

Die vorliegende Erfindung betrifft einen Wundabdeckungsartikel mit Vorbereitung zum Anschluss einer Unterdruckvorrichtung gemäß dem Oberbegriff des Anspruchs 1.

Wunddrainagesysteme zur Behandlung stark exudierender Wunden, insbesondere bei Ödemen, sind allgemein bekannt. Die einzelnen Bestandteile können je nach Funktion des Gesamtsystems variieren, wobei der generelle Aufbau im Wesentlichen gleich bleibt. Kernkomponenten sind dabei eine gas- und fluiddichte Wundabdeckung und ein Unterdruck erzeugendes Mittel, z.B. eine Vakuumpumpe. Über einen Drainageschlauch, der unterhalb des Wundabdeckungselements angeordnet sein muss, können so in der Wunde befindliche Stoffe evakuiert werden.

Solche Drainagevorrichtungen zur Wundbehandlung sind z.B. aus dem Patent EP 1 814 609 B1 bekannt. Die Vorrichtung weist einen Absorptionskörper mit superabsorbierenden Partikeln auf, der im Wundraum platziert wird. Darüber wird ein gasdichtes folienartiges Wundabdeckungselement adhäsiv am Körper des Patienten befestigt. Parallel zu dem Wundabdeckungselement wird ein Drainageschlauch in den Wundraum eingerührt, über den die im Wundraum befindliche Stoffe evakuiert werden können.

Aus der DE 29 53 373 C2 ist ebenfalls eine Vorrichtung zur Wundbehandlung unter Einsatz von Unterdruck bekannt, die aus einem Wundabdeckungselement, einer darunter liegenden Schaumstoff-Einlage und wenigstens einer mit den Poren der Schaumstoff-Einlage kommunizierenden Schlauchleitung aufgebaut ist.

Die EP 2 098 2571 betrifft einen Wundauflage mit einem Anschluss für einen Vakuumtherapie und offenbart die Verwendung von Absorptionskörpern zur Aufnahme des Wundexudats. Das Exudat wird über Perforationen in der Trägerschicht der Auflage und ein damit verbundenes Anschlussstück durch den Vakuumschlauch abgesaugt und ein einer externen Sammelflasche entsorgt.

Die WO 2009/124125 A2 betrifft eine Wundauflage mit einem normalerweise geschlossenen Ventil, das zur Entnahme der Wundflüssigkeit mittels angelegtem Vakuum geöffnet werden kann. Sie offenbart hierbei die Verwendung von Hydrokolloiden oder Hydrogelen als Absorptionskörper. De Anschluss der Unterdruckvorrichtung geschieht über eine mit einem Deckel verschließbare Öffnung. Zudem lehrt die WO '933 die Verwendung eines Septums, das als Materialverdickung des Trägermaterials ausgeführt ist.

Die WO 2009/0771933 A2 betrifft eine Vorrichtung für topische Unterdrucktherapie. Sie lehrt die Verwendung eines Schaumstoffkörpers, der in die Wunde eingelegt wird, um Wundexudat aus der Wunde zu entfernen. Zu diesem Zweck wird eine Abdeckung über die Wunde platziert, an der sich ein kuppelartiger Anschluss nebst Filter befindet.

Die WO 2010/094957 A1 betrifft eine Flüssigkeitsleitung durch eine mit einer Drapierung ausgefüllten Wundhöhle. Sie lehrt hierbei eine komplexe Anschlussvorrichtung und die Verwendung von Schaumstoff oder Mull innerhalb der Wundhöhle.

Weitere Wunddrainagesysteme mit darin angeschlossenen Vakuumpumpen sind hinreichend aus dem Stand der Technik bekannt. Solche Wunddrainagesysteme sind, wie zuvor beschrieben, in ihrem Aufbau sehr komplex und in ihren einzelnen Komponenten teuer und in der Anwendung schwierig zu handhaben. Insbesondere die darin verwendeten Wundabdeckungselemente müssen den speziellen Anforderungen an ein solches Vakuumsystem Stand halten können und sind daher oft mit erhöhten Kosten verbunden. Aufgabe der vorliegenden Erfindung ist es daher einen Wundabdeckungsartikel zum Einsatz in einem Unterdrucksystem bereit zu stellen, der einfach und flexibel in der Handhabung ist und nur geringe Herstellungskosten erfordert.

### AUSFÜRHLICHE BESCHREIBUNG DER ERFINDUNG

Diese Aufgabe wird durch den Wundabdeckungsartikel gemäß dem Hauptanspruch gelöst. Weitere bevorzugte Ausführungsformen werden durch die Merkmale der abhängigen Ansprüche wieder gegeben.

Demnach offenbart die vorliegende Erfindung einen Wundabdeckungsartikel, der ein folienartiges Element mit verminderter Gasdurchlässigkeit aufweist, wobei besagtes folienartiges Element eine Vorbereitung zum Anschluss einer Unterdruckvorrichtung aufweist.

Dabei bedeutet "folienartig", dass sich das Material des Wundabdeckungsartikels flexibel dem Körper des Patienten anpassen lässt und zur Verwendung in der Wundversorgung generell geeignet ist. Geeignet ist insbesondere eine Folie aus Polyethylen-, Polypropylen, oder Polytetrafluorethylen, oder ein Gewebe oder Vlies, das eine verminderte Gasdurchlässigkeit aufweist, beispielsweise ein Polypropylenvlies mit einer nahezu gasdichten Beschichtung, wie z.B. Polyurethan. In diesem Zusammenhang bedeutet "verminderte Gasdurchlässigkeit", dass der Wundabdeckungsartikel nicht zwingend absolut gasdicht sein muss, jedoch beim optionalen Anschluss einer Unterdruckvorrichtung dem Unterdruck Stand hält und entsprechende Fluide aus der Wunde unterhalb des Wundabdeckungselement evakuiert werden können. Das folienartige Element kann demnach auch perforiert sein, sofern es weiterhin den Anforderungen an ein angeschlossenes Vakuumsystem gerecht wird.

In diesem Zusammenhang ist es wesentlich für den erfindungsgemäßen Wundabdeckungsartikel, dass dieser sowohl passiv, d.h. als reines Wundabdeckungselement, als auch aktiv, d.h. in Verbindung mit einer Unterdruckvorrichtung verwendet werden kann, da die variabel ausgestaltete Vorbereitung zum Anschluss einer Unterdruckvorrichtung jederzeit angeschlossen werden kann. Dies hat insbesondere den Vorteil, dass erst bei einer sich abzeichnenden Ansammlung von Wundexudaten schnell und einfach zusätzlich mit Unterdruck gearbeitet werden kann, ohne dass die bereits aufgelegte Wundabdeckung ausgetauscht werden muss. Dies ist insbesondere bei stark exudierenden Wunden, insbesondere chronischen Wunden, wie sie z.B. bei Diabetes, Ulcus cruris und ähnlichen Erkrankungen besonders vorteilhaft und ermöglicht es dem Pflegepersonal die notwendige Versorgung schnell und flexibel dem Heilungsprozess der Wunde anzupassen.

Darüber hinaus bietet sich so die Möglichkeit, je nach Beschaffenheit der Wunde die passende Drainagetherapie zu wählen. Bei Wunden, bei welchen ein relativ geringer Sog ausreicht, kann der Wundabdeckungsartikel passiv betrieben werden. Ist ein relativ höherer Sog erforderlich, um die Wunde effektiv zu entwässern, kann durch Anschließen einer Unterdruckvorrichtung in einen aktiven Betriebsmodus gewechselt werden.

Die besagten Unterdruckvorrichtungen werden in der Regel mit einem atmosphärischen Unterdruck von -80 bis -150 mm Hg betrieben. In bestimmten Fällen kann jedoch bereits ein Unterdruck von -80 mm Hg zu Irritation oder sogar Schädigungen, beispielsweise durch Ischämien, führen. Daher kann es auch aus dieser Hinsicht sinnvoll sein, Wunden, die einen höheren Sog nicht unbedingt benötigen, um effektiv entwässert zu werden, einem solchen höheren Sog auch nicht auszusetzen.

Der Wundabdeckungsartikel gemäß der vorliegenden Erfindung kann, wenn er passiv verwendet wird, einen hydrodynamischen Sog von -10 bis -100 mm Hg entwickeln. Insbesondere kann der hydrodynamische Sog bei -20, -30 , -40 , -50, -60, -70, -80 oder - 90 mm Hg liegen. Durch Dosierung des Anteils an superabsorbierenden Polymeren, modifizierter Cellulose und/oder Alginaten kann dieser Sog genau eingestellt werden. So kann dieser beispielsweise zwischen 40 Gew.-% und 99 Gew.-% des Wundabdeckungsartikels betragen. Sämtliche numerischen Abstufungen zwischen diesen Werten sollen als offenbart gelten.

Dabei ist einerseits von Vorteil, dass der sich einstellende hydrodynamische Sog per se weniger Irritationen auf das Gewebe ausübt, und ferner können so bei Wunden, die die einen höheren Sog nicht unbedingt benötigen, um effektiv entwässert zu werden, die mit einer Vakuumtherapie einhergehenden Irritationen vermieden werden. Gleichzeitig bleibt die Handlungsoption eröffnet, dass für den Fall, dass die Wunde zu einem späteren Zeitpunkt einen höheren Sog benötigt, durch Zuschalten einer Unterdruckvorrichtung diesem Bedarf Rechnung getragen werden kann. Umgekehrt kann für den Fall, dass Fall, dass die Wunde zu einem späteren Zeitpunkt einen geringeren Sog benötigt, die Unterdruckvorrichtung wieder abgekoppelt werden.

Aber auch die Verwendung im Aktivmodus weist erhebliche Vorteile auf: so kann der durch die Unterdruckvorrichtung ausgeübte atmosphärische Unterdruck geringer eingestellt werden als bei einer Unterdruckvorrichtung, die ohne den erfindungsgemäßen Wundabdeckungsartikel betrieben wird, da sich im Aktivmodus der hydrodynamische Sog und der atmosphärische Unterdruck komplementieren. Dies hat positive Auswirkungen unter anderem auf die Batterielebensdauer und das Betriebsgeräusch. Ferner können durch den geringeren atmosphärischen Unterdruck die genannten Irritationen vermieden werden.

Unter "Vorbereitung" wird generell jede Art von Element des Wundabdeckungsartikels verstanden, die geeignet ist eine Unterdruckvorrichtung daran anzuschließen. Die Vorbereitung zum Anschluss einer Unterdruckvorrichtung kann somit verschieden ausgestaltet sein und eine oder mehrere der folgenden Ausgestaltungen aufweisen:
a) eine lokale Materialverdünnung,
b) eine lokale, mit einem durchstechbaren Schutzelement unter- oder überlegte Materialaussparung,
b) eine lokale, mit einem abziehbaren Klebeelement unter- oder überlegte Materialaussparung,
c) eine Markierung , die eine mögliche Durchstichstelle anzeigt,
d) eine mit einem Deckel verschließbare Öffnung, und/oder
e) einen Flansch für eine Kupplungseinrichtung.

Unter einer "lokalen Materialverdünnung" wird dabei lediglich ein Materialabschnitt im folienartigen Element verstanden, der dünner ist als das umliegende Material und somit einfacher zu durchtrennen oder zu durchstechen ist um eine Verbindung zu der Unterdruckvorrichtung herzustellen. Eine "lokale, mit einem durchstechbaren Schutzelement unter- oder überlegte Materialaussparung" bezeichnet eine runde Öffnung oder ähnlich geformte Aussparung, die mit einem Schutzelement geschlossen wird, indem die Aussparung unter- oder überlegt wird. Das Schutzelement wird seinerseits mit dem folienartigen Element verbunden und kann aus einem anderen Material als das folienartige Element sein. Als Material für das Schutzelement eignen sich alle Materialien, die wiederrum eine leichte Verbindung mit einer Unterdruckvorrichtung zulassen, insbesondere eine Kunststofffolie oder auch eine Aluminium beschichtete Folie, wie sie in ähnlicher Form für Strohhalmdurchstechöffnungen bei Trinkpäckchen verwendet wird. Das zugrunde liegende Prinzip ist dem von Trinkpäckchen generell sehr ähnlich, nämlich eine Verbindungsherstellung mittels Durchtrennen des Schutzelementes.

Alternativ zu dem durchstechbaren Schutzelement ist auch eine mit einem abziehbaren Klebelement unter- oder überlegte Materialaussparung denkbar. Dabei wird die Öffnung im folienartigen Element mit z.B. einer abziehbaren Lasche überklebt und bei Bedarf abgezogen. Sofern das Klebelement unterlegt wird, wird es vor dem Gebrauch entfernt, so dass die Öffnung für den Anschluss einer Unterdruckvorrichtung frei liegt. In einer weiteren Variante, kann das Klebelement jedoch auch selbst als Durchtrittsstelle dienen, um z.B. über eine Kanüle eine Verbindung zu der Unterdruckvorrichtung herzustellen. Gegebenenfalls lässt sich das durchstechbare Schutzelement auch mit einem darüber liegenden abziehbaren Klebelement verbinden.

Sowohl das durchstechbare Schutzelement als auch das abziehbare Klebeelement können aus ähnlichen Materialien wie das folienartige Element oder aber aus Papier, einem geeigneten Material für Membranen oder einem Verbundstoff aus Papier und beispielsweise einer metallischen Folie sein.

Bei einer vereinfachten Form der Vorbereitung weist diese lediglich eine Markierung, die eine mögliche Durchstechstelle anzeigt, auf dem folienartigen Element auf und wird somit sichtbar gemacht. Diese einfache Form lässt sich ebenfalls mit den zuvor genannten Ausführungen der Vorbereitung kombinieren.

In einer weiteren Variante weist die Vorbereitung einen Deckel auf, der eine entsprechende Öffnung zum Anschluss einer Unterdruckvorrichtung verschließt. Der Deckel kann sowohl als Einwegartikel ausgeführt sein, d.h. nach dem Öffnen nicht wieder verwendbar sein, oder aber mehrfach verwendet werden und dabei als Schraub- oder Druckdeckel, ähnlich wie bei Kunststoffbehältern, ausgeführt sein. Zusätzlich kann der Deckel über eine Lasche mit dem folienartigen Element verbunden werden, so dass bis zum Schließen der Öffnung nicht verloren geht.

In einer weiteren Variante ist die Vorbereitung ein Flansch für eine Kupplungseinrichtung, so dass die Unterdruckvorrichtung lediglich auf die Vorbereitung aufgesetzt werden muss und dadurch eine feste und dichte Verbindung hergestellt wird. Auch diese Variante kann natürlich mit einem durchstechbaren Schutzelement, aber auch mit anderen Varianten der Vorbereitung kombiniert werden.

In einer weiteren Ausführungsform des Wundabdeckungsartikels ist das folienartige Element gasdicht und/oder wasserdampfdurchlässig. Wie bereits erwähnt, ist es nicht zwingend notwendig, dass das Wundabdeckungselement komplett gasundurchlässig ist, jedoch wird die Evakuierung von Stoffen aus dem Wundraum durch ein gasdichtes folienartiges Element unterstützt, da der Unterdruck besser aufgebaut werden kann und keine Gase von außerhalb der Wunde eingesogen werden. Eine verminderte Gasdurchlässigkeit genügt jedoch um optional unter Einsatz von Unterdruck zu evakuieren. Die Wasserdampfdurchlässigkeit des folienartigen Elements ist nur dann von Bedeutung, wenn sich vermehrt Wasser in Ödemgewebe anreichert und ungehindert entweichen können soll, bevor zusätzliche Evakuierungsmittel angeschlossen werden.

In einer weiteren Ausführungsform des Wundabdeckungsartikels ist das folienartige Element zumindest abschnittsweise durchsichtig und/oder weist eine fensterartige wiederverschließbare Öffnung auf, durch welche in Gebrauch Material aus dem darunter liegendem Wundraum entnommen oder in diesen eingelegt werden kann. Dies ist insbesondere dann von Interesse, wenn das folienartige Element zusammen mit einem Absorptionskörper verwendet werden soll. Die Öffnung kann darüber hinaus mit einem Deckel ausgestattet sein und somit wieder verschlossen werden.

Erfindungsgemäß umfasst der Wundabdeckungsartikel außerdem einen Absorptionskörper, der wiederrum ein hydroaktives Polymer aufweist, welches bevorzugt ausgewählt ist aus der Gruppe enthaltend superabsorbierende Polymere, modifizierte Cellulose und/oder Alginat. Der Absorptionskörper kann sowohl fest mit dem folienartigen Element verbunden sein, oder aber lose in die Wunde eingelegt werden, bevor die eigentliche Wundabdeckung erfolgt.

Als hydroaktive Polymere kommen mehrere Stoffe in Frage: Superabsorbierende Polymere (SAP) sind Kunststoffe, die in der Lage sind, ein Vielfaches ihres Eigengewichts - bis zum 1000-fachen - an Flüssigkeiten aufzusaugen. Chemisch handelt es sich dabei um ein Copolymer aus Acrylsäure (Propensäure, C₃H₄O₂) und Natriumacrylat (Natriumsalz der Acrylsäure, NaC₃H₃O₂), wobei das Verhältnis der beiden Monomere zueinander variieren kann. Zusätzlich wird ein so genannter Kernvernetzer (Core-Cross-Linker, CXL) der Monomerlösung zugesetzt, der die gebildeten langkettigen Polymermoleküle stellenweise untereinander durch chemische Brücken verbindet (sie "vernetzt"). Durch diese Brücken wird das Polymer wasserunlöslich. Beim Eindringen von Wasser oder wässrigen Salzlösungen in den Polymerpartikel quillt er auf und strafft auf molekularer Ebene dieses Netzwerk, so dass das Wasser ohne Hilfe nicht mehr entweichen kann. Die superabsorbierenden Polymere können in dem erfindungsgemäßen Wundpflegeartikel in Form eines Granulats, eines Pulvers, einer Schüttung, eines Presslings, eines Schaums, in Form von Fasern, eines Fasergewirkes, -geleges oder -vlieses und/oder einer Faserwatte vorliegen.

Bei modifizierter Cellulose handelt es sich bevorzugt um Derivate der Cellulose, bevorzugt sulfoalkylierte Cellulose und deren Derivate, bevorzugt Celluloseethylsulfonate, carboxyalkylierte Cellulose, bevorzugt Carboxymethylzellulose, Carboxyethylcellulose und/oder Carboxypropylcellulose, komplexere Cellulosederivative, wie Sulphoethylcarboxymethylcellulose, Carboxymethylhydroxyethylcellulose, Hydroxy-propylmethylcellulose, und amidierte Cellulosederivate, wie Carboxymethylcellulose-Amid oder Carboxypropylcellulose-Amid. Carboxymethylcellulose liegt insbesonderte in Form von Natriumcarboxymethylcellulose vor und ist unter dem Namen "Hydofaser" im Handel. In Hygiene- und Wundprodukten werden die Fasern in eine flächige Matrix überführt. Durch die Aufnahme von Flüssigkeit aus dem Wundexsudat werden die Fasern nach und nach in ein Gelkissen umgewandelt, das die Flüssigkeit hält und nicht wieder freigibt. Dabei sind die Fasern so aufgebaut, dass das Wundexsudat nur in vertikaler Richtung aufgenommen wird. Dies bedeutet dass, solange die Kapazität reicht, das Exsudat nicht über den Wundrand fließt. Auf diese Weise kann eine Wundrandmazeration effektiv verhindert werden.

Bei besagten hydroaktiven Polymeren kann es sich auch um Alginate handeln. Alginate werden aus den Braunalgen gewonnen und zu einem faserigen Vlies verwoben. Chemisch handelt es sich um Polysaccharide, und zwar Calcium- und/oder Natrimsalze der Alginsäuren. Alginate können bis zum 20-fachen ihres Eigengewichtes an Flüssigkeit aufnehmen, dabei wird das Wundexsudat in die Hohlräume eingelagert. Die im Alginatgitter enthaltenen Ca2+ Ionen werden gegen die Na+ Ionen aus dem Exsudat ausgetauscht, bis der Sättigungsgrad an Na-Ionen im Alginat erreicht ist. Dabei kommt es zu einem Aufquellen der Wundauflage und zur Umwandlung der Alginatfaser in einen Gelkörper durch Aufquellen der Fasern.

Ebenso kann es sich bei besagten hydroaktiven Polymeren auch um Hydrogel-Nanopartikel aufweisend Hydroxy-Terminierte Methacrylatmonomere, wie 2-Hydroxyethylmethacrylat (HEMA) und/oder 2-Hydroxypropylmethacrylat (HPMA), die z.B. als Altrazeal vermarktet werden, handeln.

In einer weiteren bevorzugten Ausgestaltung ist vorgesehen, dass der Absorptionskörper einen Anteil von ≥ 40 Gew.-% an superabsorbierenden Polymeren aufweist. Besonders bevorzugt beträgt der Gewichtsanteil der superabsorbierenden Polymere ≥ 45, 50, 55, 60, 65 oder 70 Gew.-%. Wundpflegeartikel mit derart hohen Gewichtsanteilen an superabsorbierenden Polymeren weisen eine besonders hohe Aufnahmekapazität für Flüssigkeiten auf und können u.U. auf dem Markt befindliche Vakuum-Wundversorgungssysteme ersetzen.

Diese Ausführungsform ist besonders vorteilhaft, da ihr die Eigenschaft innewohnt, dass sie zunächst einmal wie eine normale Wundauflage verwendet werden kann ("Passiv-Modus"), beispielsweise zum Entzug von Wundexsudat aus dem Wundgrund in einer ödematösen Wundregion, wie sie beispielsweise bei Ulcus cruris oder Dekubitus auftritt.

Stellt sich heraus, dass das Absorptions- und Drainagepotential nicht ausreicht, kann über die erfindungsgemäße Vorbereitung zum Anschluss einer Unterdruckvorrichtung eine Unterdruckvorrichtung angeschlossen werden und die Ausführungsform als Vakuum-Wundversorgung weiterbetrieben werden ("Aktiv-Modus"), ohne dass die Wundauflage gewechselt werden muss, was für den Patienten einen erheblichen Zuwachs an Komfort bedeutet.

In einer weiteren bevorzugten Ausführungsform weist der Absorptionskörper mindestens ein flächiges Element aus Schaumstoff und/oder Vlies auf

Der Begriff "Schaumstoff" bezeichnet einen offen- oder geschlossenporigen Schaumstoff, bevorzugt aus Polyurethan. Der Begriff "Vlies" bezeichnet ein textiles Flächengebilde aus einzelnen Fasern, das im Gegensatz zu Geweben, Gestricken und Gewirken nicht aus Garnen hergestellt wird. Vliese behalten ihre strukturelle Integrität i.d.R. durch Haftung der einzelnen Fasern aneinander. Sie werden auch als "Nonwovens" bezeichnet, und z.B. durch Walken der Fasern hergestellt. Der Begriff "Airlaid" bezeichnet einen speziellen Vliesstoff aus Zellstoff und Polyolefinfasern, in den ggf. superabsorbierende Polymere eingebettet sind.

Der Absorptionskörper kann bevorzugt einen im Wesentlichen flachen Absorptionskörper aus Absorptionsmaterial aufweisen, der aus einem aufsaugenden Vlies mit darin verteilten superabsorbierenden Polymeren besteht. Diese können in Form eines Granulats, eines Pulvers, einer Schüttung, eines Presslings, eines Schaums, in Form von Fasern, eines Fasergewirkes, -geleges oder -vlieses und/oder einer Faserwatte vorliegen.

Dabei weist der Absorptionskörper mindestens ein Material auf, das ausgewählt ist aus der Gruppe enthaltend eine Matte, insbesondere aus einem Airlaid aus besagten Garnen oder Fasern aus superabsorbierenden Polymeren mit eingearbeiteten superabsorbierenden Polymeren, und/oder eine lose Füllung aus superabsorbierenden Polymeren. Besagte Airlaidmatte kann bevorzugt einen im Wesentlichen flachen Materialabschnitt aus Absorptionsmaterial aufweisen, der z. B. aus einem aufsaugenden Vlies aus den genannten Fasern mit darin verteilten superabsorbierenden Polymeren besteht.

Dieser Absorptionskörper kann der absorbierenden Einlage entsprechen, die in einer Wundauflage der Anmelderin der vorliegenden Erfindung enthalten ist, wie sie beispielsweise in der WO03094813, der WO2007051599 und der WO0152780 offenbart ist und unter dem Handelsnamen "sorbion sachet" vertrieben wird.

Der Absorptionskörper kann in einer anderen Ausgestaltung ebenso einen Kern bilden, der - ggf. flockenartige - Fasern oder Garne aus superabsorbierenden Polymeren sowie superabsorbierenden Polymeren in Granulatform aufweist, wobei die Granulate an die Fasern bzw. Garne in mehreren Höhen angeklebt bzw. angeschweißt sind, und die Granulate über mehr als 50 % der gesamten Bauhöhe wenigstens eines Abschnitts des Kerns verteilt sind, wobei vermengte Bereiche von Granulat und Fasern vorliegen. Der Gewichtsanteil der superabsorbierenden Polymeren kann dabei bevorzugt im Bereich zwischen 10 - 25 Gew.-% liegen. Ähnliche Konstruktionen sind aus herkömmlichen Inkontinenzmaterialien bekannt und wie Hygienebinden für ihre polsternden Eigenschaften bekannt. Um besagten Kern herum kann eine Hülle angeordnet sein, die in Bereichen überlappend angeordnet ist, und der z.B. eine Klebenaht überdeckt bzw. Teil derselben ist.

Der Absorptionskörper kann in einer anderen Ausgestaltung ebenso mindestens eine flache Lagen aufweisend Fasern oder Garne aus superabsorbierenden Polymeren enthalten, an welche superabsorbierende Polymere in Granulatform geklebt sind. Dadurch ergibt sich in einer bevorzugten Ausgestaltung ein Aufbau des Körpers, der wenigstens drei Schichten aufweist, wobei zwei Deckschichten eine Schicht aufweisend superabsorbierende Polymere umgeben.

Dabei liegen in der Ebene keine Vermengungen von Fasern und superabsorbierenden Polymeren vor; sondern lediglich fixierte Benachbarungen beider Materialien. Die ggf.

vorgesehenen mehreren Lagen können dabei in einer bevorzugten Ausgestaltung auch durch Walzen, Pressen, Kalandrieren oder ähnliche Verfahren physisch miteinander verdichtet sein. Überdies kann der Körper sich wiederholende Musterungen oder Maserungen aufweisen, wie z.B. ein Karomuster, ein Stanzmuster oder dergleichen.

In einer weiteren Ausführungsform weist der Wundpflegeartikel einen Absorptionskörper auf, der von einer äußeren Hülle umgeben ist, wobei die Hülle Mittel aufweist, die so gestaltet und/oder ausgewählt sind, dass die Hülle zumindest teilweise durch eine durch Flüssigkeitsaufnahme bedingte Volumenzunahme des Absorptionskörpers zielgerichtet verformbar ist. Die Hülle kann mindestens abschnittsweise ein integraler Bestandteil, des folienartigen Elements sein.

Vorzugsweise weist der Absorptionskörper eine Hülle auf, wobei
a) der Absorptionskörper in Draufsicht auf seine Flachseite eine Fläche (F1) aufweist, welche im nicht benetzten Zustand um 3% bis 75% kleiner als die Fläche (F2) der flachgelegten Hülle ist, und/oder
b) die Hülle mindestens abschnittsweise ein elastisches Material aufweist.

Gemäß Option a) bildet der Absorptionskörper einen Expansionsraum aus, der gewährleistet, dass der Absorptionskörper bei Flüssigkeitsaufnahme in seinem Volumen zunehmen kann und nicht durch die Hülle eingeschränkt ist.

Gemäß Option b) ist gewährleistet, dass die Hülle sich der durch Flüssigkeitsaufnahme bedingten Expansion des Absorptionskörpers nicht entgegenstellt.

Ferner ist so gewährleistet, dass bei Anlegen eines Unterdrucks der Absorptionskörper - ausreichende Flexibilität vorausgesetzt - an den Wundgrund herangezogen werden kann, was insbesondere bei tiefen Wunden erforderlich ist, um so den Kontakt mit dem aufzunehmenden Exsudat herzustellen.

Alternativ kann vorgesehen sein, dass auch oder anstelle dessen das folienartige Element mindestens abschnittsweise ein elastisches Material aufweist - mit denselben Vorteilen wie oben geschildert.

Die Poren oder Maschen der Hülle sind bevorzugt so ausgestaltet, dass sie kleiner sind als die Superabsorberpartikel. Dies ist besonders deswegen von Bedeutung, weil ansonsten Superabsorberpartikel aus dem Absorptionskörper ausrieseln können und daher von der Hülle zurückgehalten werden müssen.

Zwar unterliegt die Größe der Superabsorberpartikel gewissen stochastischen Schwankungen, aber es kann dabei vorgesehen sein, dass die Poren oder Maschen der Hülle bevorzugt kleiner als der mittlere Durchmesser besagter Superabsorberpartikel, besonders bevorzugt kleiner als der mittlere Durchmesser der einfachen oder mehrfachen Standardabweichung besagter Superabsorberpartikel sind.

Bevorzugt können die Poren z.B. 0,05 mm bis 1,0 mm, vorzugsweise 0,20 mm bis 0,50 mm gross sein. Weiterhin kann bevorzugt vorgesehen sein, dass die Poren oder Maschen durch die Fäden- oder Faserabschnitte begrenzt sind, welche im Schnitt durch die Hülle etwa bogenförmig sind und mit ihren Bogen-Scheiteln nach außen zeigen.

In einer weiteren Ausführungsform weist der Wundabdeckungsartikel an mindestens einem seiner Ränder ein flächiges Klebeelement aufweist.

Auf diese Weise kann der Artikel luftdicht an der Haut des Patienten um die zu behandelnde Wunde herum angebracht werden. Alternativ kann der Artikel mithilfe von Klebestreifen an der Haut des Patienten angebracht werden. Als Klebemittel wird ein physiologisch akzeptables Klebemittel, wie z.B. ein Hydrokolloidkleber bevorzugt. Ebenso kann auch ein Silikon- oder Acrylatklebemittel vorgesehen sein.

In einer weiteren Ausführungsform des Wundabdeckungsartikels weist die Vorbereitung einen Abstandhalter zur Tiefenbegrenzung beim Anschluss einer Unterdruckvorrichtung auf. Dieser Abstandhalter kann z.B. ein weniger Millimeter großer Zahnring sein, der unterhalb der markierten Vorbereitung eingearbeitet ist, um beim Durchstechen oder Durchschneiden der Vorbereitung zum Anschluss der Unterdruckvorrichtung nicht die darunterliegende Wunde weiter zu verletzen oder den Absorptionskörper zu treffen. Je nach Aufbau des gesamten Wundabdeckungsartikels wird somit die Tiefe in der eine Wunddrainage mit Hilfe der Unterdruckvorrichtung ansetzen soll genau vordefiniert. Eine weitere Möglichkeit die Tiefe vorab zu definieren, besteht bei der zuvor genannten Verwendung eines Kupplungselements oder eines Flansches. Bei solchen Systemen, die eine genaue Passform erfordern, ist die Höhe in der die Drainage nach erfolgter Verbindung ansetzt durch das System als solches bereits vorgegeben.

In einer weiteren Ausführungsform weist der Wundabdeckungsartikel mindestens eine weitere Lage auf, wobei das Material der Lage ausgewählt ist aus der Gruppe enthaltend
a) Wunddistanzgitter, bevorzugt dreidimensional,
b) feuchtigkeitsableitende Schicht mit hohem Trocknungspotential,
c) modifizierte Cellulose aufweisende Schicht,
d) Silber oder Silberionen aufweisende Schicht, und/oder
e) Bindungspotential für Bakterien aufweisende Schicht.

Besagte Lage wird bevorzugt an der wundzugewandten Seite angeordnet, beispielsweise unter dem optional bereit gestellten Absorptionskörper.

Bei besagtem Wunddistanzgitter handelt es sich bevorzugt um ein dreidimensionales Wunddistanzgitter wie beispielsweise in der EP2004116A1 beschrieben. Ein solches Wunddistanzgitter, das eine glatte und eine rauhe Seite aufweist, hat nicht nur eine Art Ventilfunktion für die abzuführende Flüssigkeit, sondern verhindert auch eine Eingranulation der Wunde in die Wundauflage, was einen atraumatischen Verbandswechsel erleichter. Ferner ist ein solches Material geeignet, krankheitserregende Bakterien zu binden, und es kann, wenn es mit der rauhen Seite auf die Wunde aufgebracht wird, zu einem atraumatischen Debridement sowie einer Ablösung von unerwünschten Biofilmen führen.

Alternativ kann das Wunddistanzgitter auch aus einem Silikonnetz oder einem Silikongitter bestehen.

Bei besagter feuchtigkeitsableitendet Schicht mit hohem Trocknungspotential handelt es sich beispielswiese um eine Schickt aus einem Material, dass ähnlich wie Sportfunktionskleidung in der Lage ist, Feuchtigkeit abzuleiten, um so eine Mazeration zu verhindern. Ein solches Material kann beispielsweise aus Polyesterfasern oder aus Fasern aufweisend merzerisierte Baumwolle bestehen.

Modifizierte Cellulosen sind z.B. Carboxymethlcellulosen (CMC) und als solches Celluloseether. Je nach Grad der Veretherung kann aus CMC eine Hydrokolloidmatrix entstehen, die als zusätzliche Schutzschicht dient.

Bei einer Silber oder Silberionen aufweisenden Schicht, stehen vor allem die antimikrobiellen Eigenschaften im Vordergrund. Erwiesenermaßen hat Silber bakteriostatische Eigenschaften, die zur Heilung der Wunde beitragen können.

Eine ein Bindungspotential für Bakterien aufweisende Schicht besteht bevorzugt aus einem hydrophoben Material, das durch Interaktionen mit der bakteriellen Zelloberfläche letztere bindet. Solche Schichten sind z.B. hinreichend aus chromatographischen Verfahren bekannt.

In einem weiteren Aspekt betrifft die vorliegende Erfindung eine Kupplungseinrichtung zum Anschluss einer Unterdruckvorrichtung an den erfindungsgemäßen Wundabdeckungsartikel.

Besagte Kupplungseinrichtung ist so ausgestaltet, dass sie über die seitens des folienartigen Elements des Wundabdeckungsartikels bereitgestellte Vorbereitung den Anschluss einer Unterdruckvorrichtung ermöglicht.

In einer bevorzugten Ausführungsform weist die Kupplungseinrichtung mindestens ein Element auf, dass ausgewählt ist aus der Gruppe enthaltend:
a) eine Kanüle (49b) zum Durchstechen eines Schutzelements und/oder einer Materialverdünnung
b) eine Klinge (49c) zum Durchstechen eines Schutzelements und/oder einer Materialverdünnung
c) ein Flanschgegenstück (33/43)
d) eine aufklebbare Kupplung (48), und/oder
e) ein Vakuumelement zur Vakuumunterstützen Befestigung der Kupplungseinrichtung an dem Wundabdeckungsartikel.

Alternativ zu der Kanüle kann jede Art einer Hohlnadel verwendet werden, die sich für den Einsatz in einer entsprechenden Kupplungseinrichtung eignet.

Bevorzugt weist die Kupplungseinrichtung eine Klinge auf, die beispielsweise federbelastet ist und beim Aufsetzen auf den Wundabdeckungsartikel durch Betätigung einen Schlitz oder ein Loch in die Vorbereitung zum Anschluss einer Unterdruckvorrichtung einbringt. Alternativ kann die Klinge in einer Kuppel, beispielswiese einer Gummikuppel, angeordnet sein, so dass sich durch Drücken auf die Kuppel ein Schlitz oder ein Loch in die Vorbereitung zum Anschluss einer Vakuumeinrichtung einbringen lässt.

Bei einem Flanschsystem erfolgt eine Kupplungsverbindung z.B. über einen Schraub- oder Bajonettverschluss.

Bei einer aufklebbaren Kupplung wird das Kupplungsgegenstück auf die entsprechende Verbindungseinheit der Vorbereitung auf dem folienartigen Element aufgeklebt. Auf dem folienartigen Element wird dazu z.B. ein Klebeelement eingesetzt, das zur Verbindungsherstellung mit der Kupplung abgezogen wird, so dass eine Öffnung freigegeben wird.

In einer alternativen Ausführungsform ist die Kupplungsvorrichtung selbst schon vakkuumunterstützt, d.h. Verbindungsherstellung erfolgt mithilfe von Vakuum, so dass durch den Unterdruck eine Kupplungsverbindung zum Wundabdeckungsartikel bzw. das folienartige Element entsteht.

In einem weiteren Aspekt betrifft die vorliegende Erfindung ein Kit zur Wundversorgung, aufweisend einen Wundabdeckungsartikel wie zuvor beschrieben sowie eine Kupplungseinrichtung wie zuvor beschrieben.

Kit-Systeme haben insbesondere den Vorteil, dass sie als Komplettsystem dem Notfall- oder Pflegepersonal zur Verfügung gestellt werden können und sich diese keine Gedanken um die passenden Einzelkomponenten machen müssen.

In einer besonderen Ausführungsform dieses Aspekts weist das Kit-System mindestens ein weiteres Element ausgewählt aus der folgenden Gruppe auf:
a) Vakuumpumpe zur Erzeugung eines Unterdrucks,
b) Schlauch, vorzugsweise Vakuumschlauch, zum Anschluss der Kupplungseinrichtung an die Vakuumpumpe,
c) Filtermembran, die den Gasdurchtritt in den Schlauch ermöglicht, den Flüssigkeitsdurchtritt jedoch verwehrt,
d) Rückschlagventil, das einen Rückfluss von evakuierter Flüssigkeit verhindert,
e) Kupplung, die das Abkoppeln des Kits von einer Vakuumpumpe ermöglicht, ohne dass das Vakuum verloren geht.

Besagte Vakuumpumpe ist bevorzugt eine miniaturisierte Pumpe, beispielsweise auf Basis eines Piezo-Pumpensystems. Wie geschildert ist die Pumpe bevorzugt abnehmbar ausgestaltet, so dass Sie vom Patienten beispielsweise in der Tasche mitgeführt und nur bei Bedarf an die Kupplungseinrichtung angeschlossen werden muss. Zu diesem Zweck kann der Wundabdeckungsartikel einen Drucksensor aufweisen, der signalisiert, ob und wenn ja wann es Zeit ist, die Pumpe anzuschließen und erneut Luft zu evakuieren. Dies kann beispielsweise erfolgen durch eine Farbcode am Sensor, oder aber durch eine Funkmitteilung, die beispielsweise an die Pumpe gesandt wird.

Alternativ kann vorgesehen sein, dass es sich bei der Vakuumpumpe um eine Einweg- oder Mehrwegspritze handelt.

In einem weiteren Aspekt betrifft die vorliegende Offenbarung die Verwendung eines Wundabdeckungsartikels wie zuvor beschrieben, einer Kupplungseinrichtung wie zuvor beschrieben und/oder eines Kits wie zuvor beschrieben, zur Behandlung von Wunden, insbesondere Wunden aufweisend tiefsitzende Ödeme.

Diese Art der Verwendung ist vorzugsweise in der Geriatrie, aber auch bei postoperativer Wund- bzw. Ödembehandlungen anzutreffen.

In einer weiteren Ausführungsform erfolgt die Verwendung eines Wundabdeckungsartikels wie zuvor beschrieben, einer Kupplungseinrichtung wie zuvor beschrieben und/oder eines Kits wie zuvor beschrieben in einem Vakuum-Wundversorgungssystem.

### Abbildungen

Die vorliegende Erfindung wird durch die im Folgenden gezeigten und diskutierten Figuren genauer erläutert. Dabei ist zu beachten, dass die Figuren nur beschreibenden Charakter haben und nicht dazu gedacht sind, die Erfindung in irgendeiner Form einzuschränken.

### Bezugszeichenliste:

- 10: Wundabdeckungsartikel
- 11/31/61: folienartiges Element
- 12: Vorbereitung
- 21/30: Klebeelement
- 22: Klebestreifen
- 32/42: Flansch
- 33/43/53: Flanschgegenstück
- 34/64: Absorptionskörper
- 35: Wunde
- 41/63: Materialaussparung
- 44/62: abziehbares Klebelement
- 45: durchstechbares Schutzelement
- 46: Schutzfolie
- 47: Klebefläche
- 48: aufklebbare Kupplung
- 49b: Kanüle oder Hohlnadel
- 49c: Klinge
- 51: Deckel
- 52: Öffnung
- 54: lokale Materialverdünnung
- 65: weitere Lage
- 66: Öffnung im folienartigen Element
- 67: Deckel zur Entnahme oder Einbringen des Absorptionskörpers
- 71: Abstandhalter
- 72: Flansch mit durchstechbarem Schutzelement
- 74: Klebeelement
- 80: Absorptionskörper
- 81: elastisches folienartiges Element
- 82: elastische Hülle
- 83: tiefe Wunde
- 84: Wundgrund
- 85: Exsudat
- 86: Kupplungseinrichtung

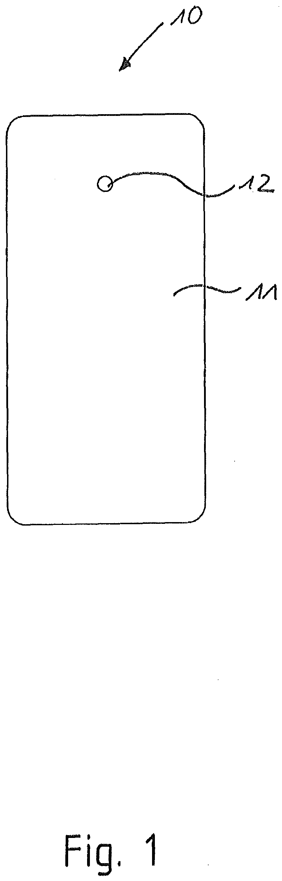

Fig. 1 zeigt den Wundabdeckungsartikel 10 in der einfachsten "Passiv-Form", d.h. mit einem folienartigen Element 11, das eine Vorbereitung 12 zum Anschluss einer Unterdruckvorrichtung aufweist und als solches auch als einfache Wundabdeckung ohne den Anschluss einer Unterdruckvorrichtung, also passiv, verwendet werden kann.

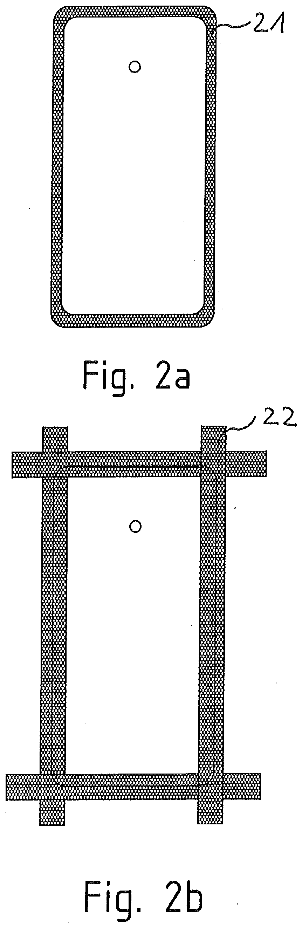

Fig. 2a zeigt den Wundabdeckungsartikel in der einfachsten Form mit einem zusätzlichen Klebeelement 21 am folienartigen Element zum Anbringen an die Haut eines Patienten. Fig. 2b zeigt den Wundpflegeartikel mit Klebestreifen 22 um das folienartige Element zum Anbringen an die Haut eines Patienten.

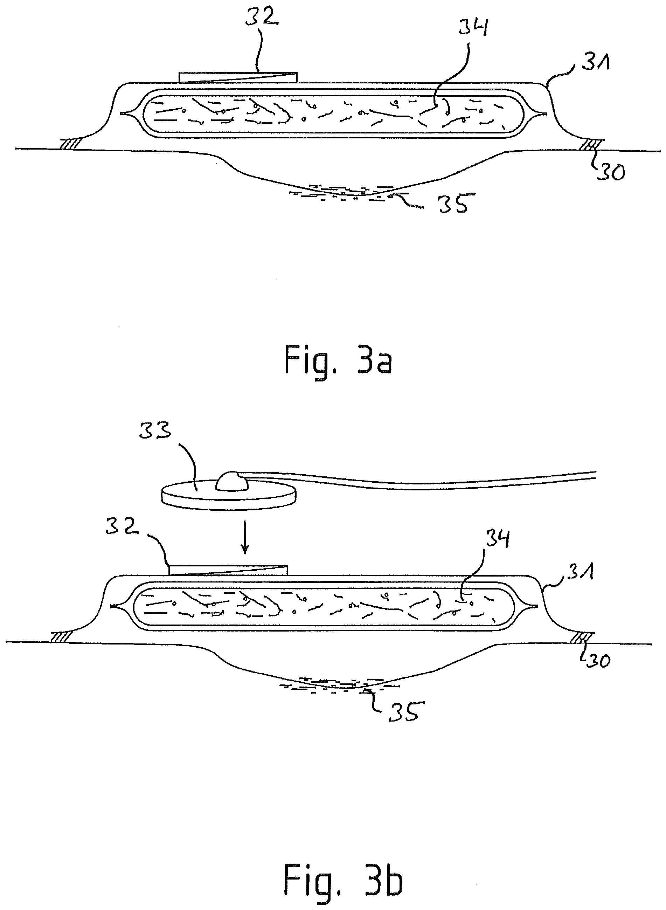

Fig. 3a zeigt den Wundabdeckungsartikel mit dem folienartigen Element 31 und einem Klebelement 30 mit dem das folienartige Element an der Haut des Patienten, die die Wunde 35 umgibt, angebracht ist. Das folienartige Element verfügt hier über einen Flansch 32 als Vorbereitung zum Anschluss einer Unterdruckvorrichtung. Zwischen dem folienartigen Element 31 und der Wunde 35 ist außerdem ein Absorptionskörper 34 in einer Hülle platziert.

Fig. 3b zeigt wie das Flanschgegenstück 33 an den Flansch 32 gekoppelt wird (s. Pfeilrichtung). Von dem Flanschgegenstück führt eine Schlauchverbindung zu der nicht gezeigten Unterdruckvorrichtung.

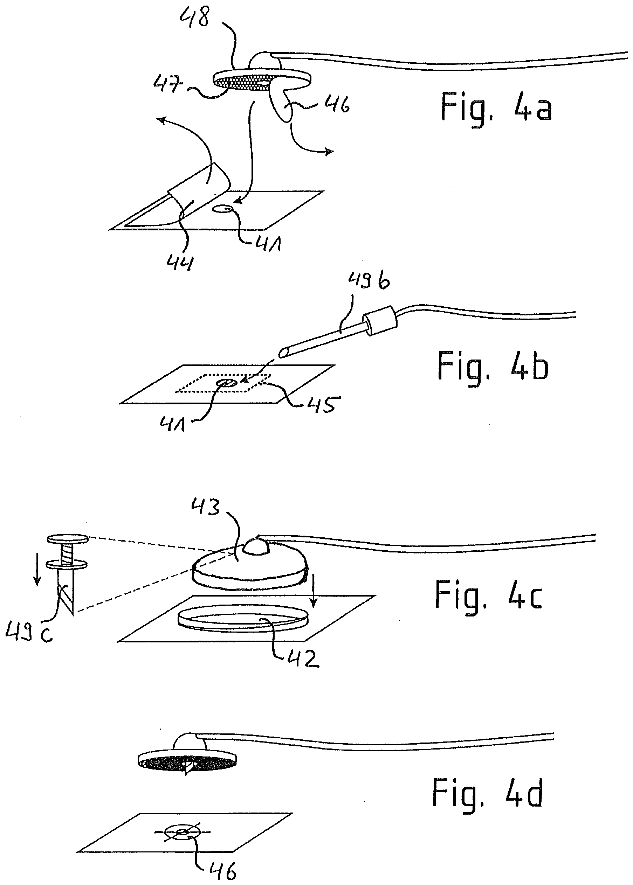

Fig. 4a bis d zeigen verschiedene Varianten der Vorbereitung auf dem folienartigen Element und mögliche darauf abgestimmter Kupplungseinrichtungen. Fig. 4a zeigt eine Vorbereitung zum Anschluss einer Unterdruckvorrichtung mit einer Materialaussparung 41 in dem folienartigen Element über das ein abziehbares Klebelement 44 gelegt ist. Zum Öffnen der Vorbereitung wird das Klebelement 44 in Pfeilrichtung abgezogen, so dass die Materialaussprung 41 nunmehr frei liegt. Die Kupplungseinrichtung 47 zum Anschluss einer Unterdruckvorrichtung weist eine Klebefläche 47 mit einer Schutzfolie 46 auf, die ebenfalls zum Anschluss an das folienartige Element abgezogen wird, so dass die Klebefläche 47 mit dem folienartigen Element rund um die Materialaussparung 41 verbunden wird. Die Verbindung zur Unterdruckvorrichtung ist somit über die aufklebbare Kupplung 48 hergestellt. An die aufklebbare Kupplung 48 schließt sich eine Schlauchverbindung an die zum nicht gezeigten Unterdruck erzeugenden Mittel führt. Fig. 4b zeigt eine weitere Variante der Vorbereitung, bei der die Materialaussparung 41 in dem folienartigen Element mit einem durchstechbaren Schutzelement 45 unterlegt ist. Als Kupplungseinrichtung wird eine Kanüle oder Hohlnadel 49b verwendet, mit der die Verbindung zur Unterdruckvorrichtung mittels Durchstechen des Schutzelementes 45 hergestellt wird. Fig. 4c zeigt eine Vorbereitung als Flansch 42 auf dem folienartigen Element. Das Flanschgegenstück 43 beinhaltet in der kleinen Gummikuppel eine gefederte Klinge 49c die mit dem Anschluss des Flanschgegenstückes 43 durch manuellen Druck auf die Kuppel runter gedrückt werden kann um so innerhalb des Flansches 42 ein Loch in das folienartige Element zu schneiden. Über den ebenfalls in der Gummikuppel endenden Schlauch wird eine Verbindung zu der nicht gezeigten Unterdruckvorrichtung geschaffen.Fig. 4d zeigt eine Markierung 46 auf dem folienartigen Element auf das die Kupplungseinrichtung mit einer Kanüle, Hohlnadel oder Klinge zum Durchstechen der Markierung und somit des folienartigen Elementes gesetzt wird.

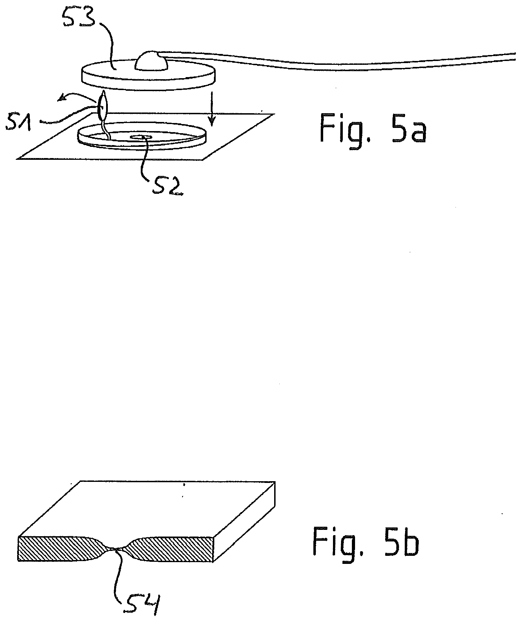

Fig. 5a zeigt noch eine weitere Variante der Vorbereitung, bei der eine Öffnung 52 innerhalb eines Flansches auf dem folienartigen Element mit einem Deckel 51 verschlossen werden kann. Durch Anheben des z.B. als Druckknopf ausgeführten Deckels 51, der ganz abgenommen werden kann oder aber mit dem Flansch mit einer kleinen Sicherung verbunden bleibt, wird die Öffnung 52 frei gegeben und das Flanschgegenstück 53 kann zur Verbindungsherstellung angeschlossen werden. Fig. 5b zeigt ein folienartiges Element mit einer lokalen Materialverdünnung 54 im Querschnitt.

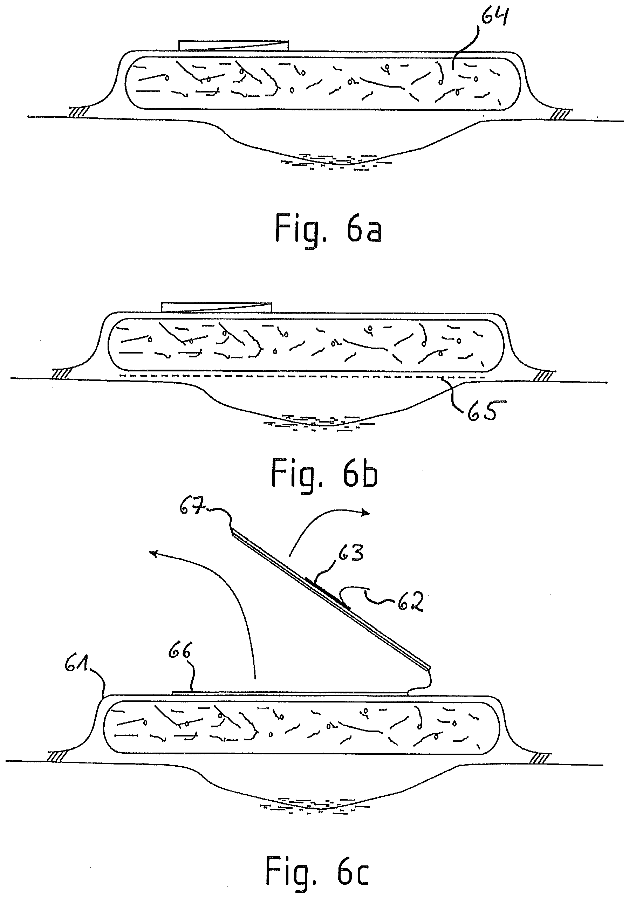

Fig. 6a zeigt einen Wundabdeckungsartikel mit einem folienartigen Element samt Vorbereitung und einem darunter liegendem Absorptionskörper 64. Fig. 6b zeigt einen Wundabdeckungsartikel mit einem folienartigen Element samt Vorbereitung und einem darunter liegendem Absorptionskörper. Unter dem Absorptionskörper ist eine weitere Lage 65 innerhalb des Wundabdeckungsartikels angeordnet. Fig. 6c zeigt einen Wundabdeckungsartikel mit einem folienartigen Element 61 und einer Öffnung im folienartigen Element 66, durch das wie durch ein Fenster der Absorptionskörper eingebracht oder entfernt werden kann. Der Deckel 67 zum Öffnen des folienartigen Elements, durch das der Absorptionskörper eingebracht oder entfernt werden kann, verfügt außerdem über eine Materialaussparung 63, die mit einem abziehbaren Klebeelement 62 bis zum Anschluss einer Unterdruckvorrichtung geschlossen bleibt.

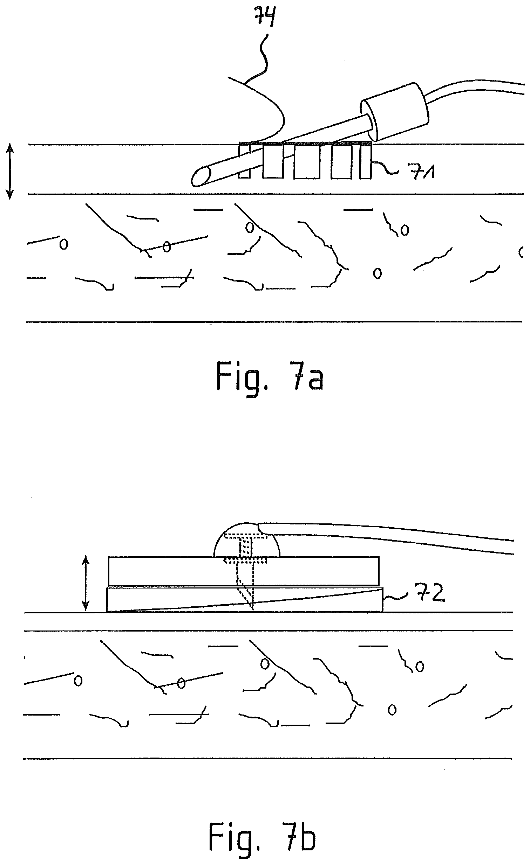

Fig. 7a zeigt einen zahnradförmigen Abstandhalter 71, der unterhalb des folienartigen Elements an der Vorbereitung angebracht ist und verhindert, dass die Hohlnadel oder Kanüle bei abgehobenem Klebeelement 74 bis in den Absorptionskörper sticht, da diese durch den Abstandhalter 71 daran gehindert wird. Sofern die Drainage bis in den Absorptionskörper reichen soll, kann ggf. auf einen solchen Abstandhalter 71 verzichtet werden.

Fig. 7b zeigt, wie durch das Flanschsystem selbst ein Sicherheitsabstand zum Absorptionskörper geschaffen wird, da der Flansch 72 die Eindringtiefe des Messers oder der Kanüle im Flanschgegenstück limitiert. Durch leichten manuellen Druck auf die Gummikuppel des Flanschgegenstückes dringt die Klinge oder Kanüle durch das folienartige Element, aber nicht bis zum Absorptionskörper vor.

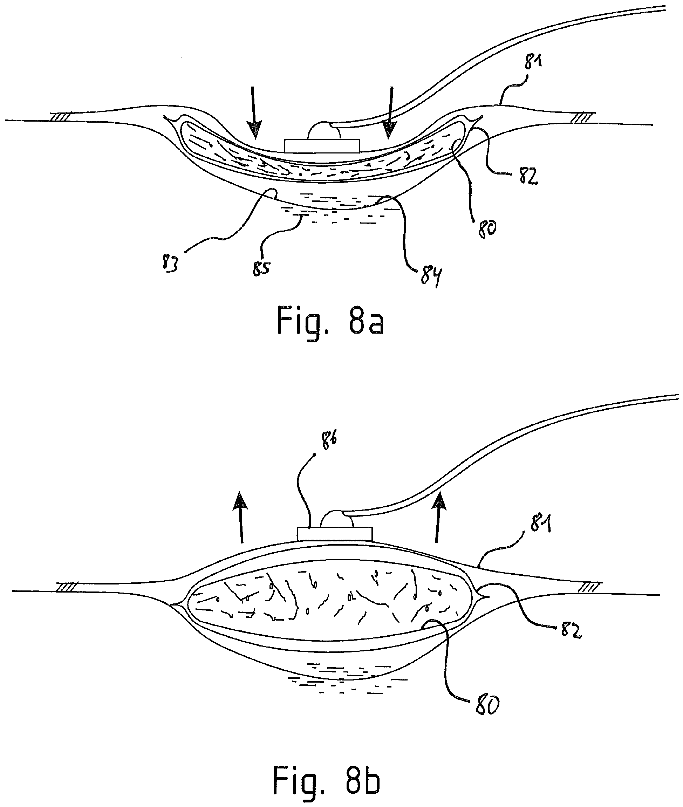

Fig. 8a zeigt die Anordnung des erfindungsgemäßen Wundpflegeartikels mit einem Absorptionskörper 80 mit einem elastischen folienartigen Element 81 und/oder einer elastischen Hülle 82 an einer tiefen Wunde 83, an deren Wundgrund 84 Exsudat 85 steht. Abweichend von Fig. 8 kann die Hülle 82 mindestens abschnittsweise - beispielsweise im wundabgewandten Bereich - ein integraler Bestandteil des folienartigen Elements sein. Durch das elastische folienartige Element 81 und/oder die elastische Hülle 82 ist gewährleistet, dass bei Anlegen eines Unterdrucks der Absorptionskörper an den Wundgrund 84 herangezogen bzw. herangepresst werden kann (angedeutet durch die Pfeile), was insbesondere bei tiefen Wunden erforderlich ist, um so den Kontakt mit dem aufzunehmenden Exsudat 85 herzustellen.

Fig. 8b zeigt eine ähnliche Anordnung wie Fig. 8a, jedoch zu einem späteren Zeitpunkt. Der Absorptionskörper 80 hat bereits große Mengen an Exsudat aufgenommen. Durch das elastische folienartige Element 81 und/oder die elastische Hülle 82 ist gewährleistet, letztere sich der durch die Flüssigkeitsaufnahme bedingten Expansion des Absorptionskörpers nicht entgegenstellen. So wird gewährleistet, dass der Absorptionskörper seine volle Aufnahmekapazität ausspielen kann. Zu diesem Zeitpunkt kann die Kupplungseinrichtung 86 (und damit die nicht dargestellte, daran angeschlossene Unterdruckvorrichtung) bereits abgekoppelt sein, oder aber die Unterdruckvorrichtung ist abgestellt und zieht keinen Unterdruck mehr.

Es kann also vorgesehen sein, dass das Anlegen eines Unterdrucks lediglich dazu dient, den Absorptionskörper an den Wundgrund 84 heranzuziehen bzw. heranzupressen, um so den Kontakt mit dem aufzunehmenden Exsudat herzustellen. Sobald dieser Kontakt hergestellt ist, kann vorgesehen sein, die Vakuumvorrichtung abzutrennen bzw. abzuschalten. In diesem Falle erfolgt die weitere Akquise von Exsudat ausschließlich durch den Absorptionskörper.

## Patentansprüche

1. Wundabdeckungsartikel (10), aufweisend:
a. ein folienartiges Element (11) mit verminderter Gasdurchlässigkeit wobei besagtes folienartiges Element eine Vorbereitung (12) zum Anschluss einer Unterdruckvorrichtung aufweist, und wobei der Wundabdeckungsartikel einen Absorptionskörper, aufweisend ein hydroaktives Polymer, ausgewählt aus der Gruppe enthaltend superabsorbierende Polymere, modifizierte Cellulose oder Alginate, umfasst;
b. sowie eine Kupplungseinrichtung zum Anschluss einer Unterdrucksvorrichtung an diesen Wundabdeckungsartikel, wobei die Kupplungseinrichtung eine Kanüle (49b), eine Klinge (49c) oder eine Hohlnadel zum Durchstechen des der Materialverdünnung aufweist,
**dadurch gekennzeichnet, dass** die besagte Vorbereitung eine lokale, durchstechbare oder durchtrennbare Materialverdünnung aufweist.

2. Wundabdeckungsartikel gemäß Anspruch 1, **dadurch gekennzeichnet, dass** das folienartiges Element gasdicht und/oder wasserdampfdurchlässig ist.

3. Wundabdeckungsartikel gemäß einem der vorherigen Ansprüche, wobei das folienartige Element zumindest abschnittsweise durchsichtig ist oder eine fensterartige Öffnung aufweist, durch welche in Gebrauch Material aus dem darunter liegenden Wundraum entnommen oder in diesen eingelegt werden kann.

4. Wundabdeckungsartikel gemäß einem der vorherigen Ansprüche, wobei der Absorptionskörper mindestens ein flächiges Element aus Schaumstoff und/oder Vlies aufweist.

5. Wundabdeckungsartikel gemäß einem der vorherigen Ansprüche, wobei der Absorptionskörper eine Hülle aufweist, und wobei
a) der Absorptionskörper in Draufsicht auf seine Flachseite eine Fläche (F1) aufweist, welche im nicht benetzten Zustand um 3% bis 75% kleiner als die Fläche (F2) der flachgelegten Hülle ist, und/oder
b) die Hülle mindestens abschnittsweise ein elastisches Material aufweist.

6. Wundabdeckungsartikel gemäß einem der vorherigen Ansprüche, wobei der Wundabdeckungsartikel an mindestens einem seiner Ränder ein flächiges Klebeelement (21) aufweist.

7. Wundabdeckungsartikel gemäß einem der vorherigen Ansprüche, wobei die Vorbereitung einen Abstandhalter (71) zur Tiefenbegrenzung beim Anschluss einer Unterdruckvorrichtung aufweist.

8. Wundabdeckungsartikel gemäß einem der vorherigen Ansprüche, wobei der Wundabdeckungsartikel mindestens eine weitere Lage (65) aufweist, und wobei das Material der Lage ausgewählt ist aus der Gruppe enthaltend
a) Wunddistanzgitter, bevorzugt dreidimensional,
b) feuchtigkeitsableitende Schicht mit hohem Trocknungspotential,
c) modifizierte Cellulose aufweisende Schicht,
d) Silber oder Silberionen aufweisende Schicht, oder
e) Bindungspotential für Bakterien aufweisende Schicht.

9. Wundabdeckungsartikel gemäß einem der vorherigen Ansprüche, wobei die Kupplungseinrichtung eine aufklebbare Kupplung (48), oder ein Vakuumelement zur vakuumunterstützenden Befestigung der Kupplungseinrichtung an dem Wundabdeckungsartikelaufweist.

10. Kit zur Wundversorgung, aufweisend einen Wundabdeckungsartikel gemäß einem der Ansprüche 1 bis 9.

11. Kit gemäß Anspruch 10, ferner aufweisend mindestens ein Element ausgewählt aus der Gruppe enthaltend:
a) Vakuumpumpe zur Erzeugung eines Unterdrucks,
b) Schlauch, vorzugsweise Vakuumschlauch, zum Anschluss der Kupplungseinrichtung an die Vakuumpumpe,
c) Filtermembran, die den Gasdurchtritt in den Schlauch ermöglicht, den Flüssigkeitsdurchtritt jedoch verwehrt,
d) Rückschlagventil, das einen Rückfluss von evakuierter Flüssigkeit verhindert,
e) Kupplung, die das Abkoppeln des Kits von einer Vakuumpumpe ermöglicht, ohne dass das Vakuum verloren geht.

12. Wundabdeckungsartikel gemäß einem der Ansprüche 1 bis 9, oder ein Kit gemäß einem der Ansprüche 10 oder 11, zur Verwendung bei der Behandlung von Wunden, insbesondere Wunden aufweisend tiefsitzende Ödeme.

13. Wundabdeckungsartikel gemäß einem der Ansprüche 1 bis 9, oder ein Kit gemäß einem der Ansprüche 10 oder 11, zur Verwendung in einem Vakuum-Wundversorgungssystem.

## Claims

1. A wound-covering article (10), comprising:
a) a film-like element (11) with reduced gas permeability, whereby said film-like element has a preparation (12) for attachment of a vacuum device, and whereby the wound-covering article comprises an absorptive element that contains a hydroactive polymer selected from the group containing superabsorbent polymers, modified cellulose or alginates;
b) as well as a coupling means for attachment of a vacuum device to this wound-covering article, whereby the coupling means has a cannula (49b), a blade (49c) or a hollow needle to puncture the thinned area of material,
**characterized in that** said preparation has a local thinned area of material that can be punctured or severed.

2. The wound-covering article according to claim 1, **characterized in that** the film-like element is gas-tight and/or water vapor-permeable.

3. The wound-covering article according to one of the preceding claims, whereby the film-like element is transparent, at least in certain sections, or it has a window-like opening through which material can be removed from or placed into the underlying wound region.

4. The wound-covering article according to one of the preceding claims, whereby the absorptive element has at least one flat element made of foam and/or a nonwoven.

5. The wound-covering article according to one of the preceding claims, whereby the absorptive element has a sheath, and whereby
a) in a top view of the flat side of the absorptive element, it has a surface area (F1) that, in the unwetted state, is 3% to 75% smaller than the surface area (F2) of the sheath laid flat, and/or
b) the sheath comprises an elastic material, at least in certain sections.

6. The wound-covering article according to one of the preceding claims, whereby the wound-covering article has a flat adhesive element (21) on at least one of its edges.

7. The wound-covering article according to one of the preceding claims, whereby the preparation comprises a spacer (71) to limit the depth during the attachment of a vacuum device.

8. The wound-covering article according to one of the preceding claims, whereby the wound-covering article has at least another layer (65), and whereby the material of the layer is selected from the group containing
a) a wound spacer lattice, preferably three-dimensional,
b) a moisture-draining layer with a high drying potential,
c) a layer containing modified cellulose,
d) a layer containing silver or silver ions, or
e) a layer having a binding potential for bacteria.

9. The wound-covering article according to one of the preceding claims, whereby the coupling means has an adhesive coupling (48) or a vacuum element for the vacuum-assisted attachment of the coupling means to the wound-covering article.

10. A kit for wound care, having a wound-covering article according to one of claims 1 to 9.

11. The kit according to claim 10, further comprising at least one element selected from the group containing:
a) a vacuum pump for generating a vacuum,
b) a hose, preferably a vacuum hose, for attachment of the coupling means to the vacuum pump,
c) a filter membrane that permits gas to enter the hose, but that repels fluid,
d) a non-return valve that prevents a backflow of evacuated fluid,
e) a coupling that permits the kit to be uncoupled from a vacuum pump without losing the vacuum.

12. The wound-covering article according to one of claims 1 to 9, or a kit according to one of claims 10 or 11, for use in the treatment of wounds, especially of wounds involving deep edema.

13. The wound-covering article according to one of claims 1 to 9, or a kit according to one of claims 10 or 11, for use in a vacuum wound care system.

## Revendications

1. Article de recouvrement de plaie (10) comportant :
a. un élément pelliculaire (11) à perméabilité aux gaz réduite, ledit élément folliculaire présentant une préparation (12) pour raccorder un dispositif de sous-pression, et l'article de recouvrement de plaie comprenant un corps d'absorption avec un polymère hydroactif choisi dans le groupe comprenant les polymères superabsorbants, la cellulose modifiée ou les alginates et
b. un dispositif d'accouplement pour raccorder un dispositif de sous-pression audit article de recouvrement de plaie, le dispositif d'accouplement présentant une canule (49b), une lame (49c) ou une aiguille creuse pour percer l'amincissement de matière,
**caractérisé en ce que** ladite préparation présente un amincissement de matière local, perçable ou sécable.

2. Article de recouvrement de plaie selon la revendication 1, **caractérisé en ce que** l'élément pelliculaire est étanche aux gaz et/ou perméable à la vapeur d'eau.

3. Article de recouvrement de plaie selon l'une des revendications précédentes, l'élément pelliculaire étant au moins en partie transparent ou présentant une ouverture de type fenêtre permettant, pendant l'usage, de retirer de la matière de l'espace de plaie situé en dessous ou d'y en introduire.

4. Article de recouvrement de plaie selon l'une des revendications précédentes, le corps d'absorption comportant au moins un élément plan en mousse et/ou en non-tissé.

5. Article de recouvrement de plaie selon l'une des revendications précédentes, le corps d'absorption présentant une enveloppe et
a) le corps d'absorption présentant, en vue de dessus, sur son côté plat, une surface (F1) qui, à l'état non mouillé, est de 3% à 75% inférieure à la surface (F2) de l'enveloppe posée à plat et/ou
b) l'enveloppe comportant au moins un partie une matière élastique.

6. Article de recouvrement de plaie selon l'une des revendications précédentes, l'article de recouvrement de plaie un élément adhésif plan (231) sur au moins l'un de ses bords.

7. Article de recouvrement de plaie selon l'une des revendications précédentes, la préparation comportant un espaceur (71) pour limiter la profondeur lors du raccordement d'un dispositif de sous-pression.

8. Article de recouvrement de plaie selon l'une des revendications précédentes, l'article de recouvrement de plaie comportant au moins une couche supplémentaire (65) et la matière de la couche étant choisie dans le groupe contenant :
a) grille d'espacement de la plaie, de préférence tridimensionnelle;
b) une couche d'évacuation de l'humidité avec un potentiel de séchage élevé ;
c) une couche comportant de la cellulose modifiée ;
d) une couche comportant de l'argent ou des ions d'argent ou
e) une couche comportant un potentiel de liaison de bactéries.

9. Article de recouvrement de plaie selon l'une des revendications précédentes, le dispositif d'accouplement présentant un accouplement à coller (48) ou un élément sous vide pour la fixation, avec assistance par vide, du dispositif d'accouplement sur l'article de recouvrement de plaie.

10. Kit pour le traitement de plaies, comportant un article de recouvrement de plaie selon l'une des revendications 1 à 9.

11. Kit selon la revendication 10, comportant en outre au moins un élément choisi dans le groupe contenant :
a) une pompe à vide pour générer une sous-pression ;
b) un tuyau flexible, de préférence un tuyau flexible sous vide, pour raccorder le dispositif d'accouplement à la pompe à vide ;
c) une membrane de filtration qui permet le passage de gaz dans le tuyau flexible, mais empêche le passage de liquide ;
d) un clapet anti-retour qui empêche un reflux de liquide évacué ;
e) un accouplement qui permet de découpler le kit d'une pompe à vide sans perte du vide.

12. Article de recouvrement de plaie selon l'une des revendications 1 à 9 ou kit selon l'une des revendications 10 ou 11 pour utilisation lors du traitement de plaies, en particulier de plaies présentant des oedèmes profonds.

13. Article de recouvrement de plaie selon l'une des revendications 1 à 9 ou kit selon l'une des revendications 10 ou 11 pour utilisation dans un système de traitement de plaie à vide.
